# EUROPEAN PATENT APPLICATION

(11) **EP 4 397 305 A1**
(43) Date of publication of application: **10.07.2024**
(21) Application number: 23150423.4
(22) Date of filing: 05.01.2023
(51) Int. Cl.: A61K 31/18, A61K 31/4164, A61K 31/421, A61K 31/426, A61K 31/573, A61P 17/00, A61P 27/02, A61P 37/00

(54) **CXCL8 INHIBITORS FOR USE IN THE TREATMENT OF OCULAR MUCOUS MEMBRANE PEMPHIGOID AND/OR ORAL MUCOUS MEMBRANE PEMPHIGOID**

(71) Applicant: Dompe' Farmaceutici S.P.A., 20122 Milan (IT)
(72) Inventor: SCHMIDT, Enno, 23538 Lübeck (DE); LUDWIG, Ralf, 23538 Lübeck (DE); PATZELT, Sabrina, 23538 Lübeck (DE); ARAMINI, Andrea, 67100 L'Aquila (IT); BRANDOLINI, Laura, 67100 L'Aquila (IT); COCCHIARO, Pasquale, 67100 L'Aquila (IT); BIANCHINI, Gianluca, 67100 L'Aquila (IT); DETTA, Nicola, 80131 Napoli (IT)
(74) Representative: Dompé farmaceutici Spa

(57) **Abstract**

The invention relates to CXCL8 inhibitors useful in the prevention and/or treatment of ocular mucous membrane pemphigoid and/or oral mucous membrane pemphigoid.

## Description

### TECHNICAL FIELD

The invention relates to CXCL8 inhibitors useful in the prevention and/or treatment of ocular mucous membrane pemphigoid (OcMMP) and/or oral mucous membrane pemphigoid.

### BACKGROUND OF THE INVENTION

Mucous membrane pemphigoid (MMP) is a systemic cicatrizing autoimmune disease that primarily affects orificial mucous membranes, such as the conjunctiva, the nasal cavity, the oropharynx, and the genitalia.

About 75% of MMP patients generate antibodies against BP180 (type XVII collagen) and 25% of MMP patients against laminin 332. In less than 5% of MMP patients, antibodies against type VII collagen or α6β4 integrin are detected (Domloge-Hultsch, N., et al., J Clin Invest, 1992. 90(4): p. 1628-33; Oyama, N., et al., Br J Dermatol, 2006. 154(1): p. 90-8; Schmidt, E., et al., Br J Dermatol, 2001. 145(5): p. 778-83).

Ocular involvement occurs in about 70% of all MMP cases and ocular MMP is the leading cause of cicatrizing conjunctivitis in developed countries.

Linear immunoglobulin A disease, mucosal dominated epidermolysis bullosa acquisita, and anti-laminin 332/anti-epiligrin/anti-laminin 5 pemphigoid are encompassed by ocular MMP (OcMMP).

The progressive inflammatory and scarring nature of ocular MMP leads to severe visual impairment in 30% of affected eyes and bilateral blindness in 20%.

Ocular MMP in often associated with oral mucosa lesions including desquamative gingivitis, vesicles, erosions covered by pseudomembranes and ulcers.

In some cases of MMP, only the oral mucosa is involved.

The underlying pathophysiological mechanism of the disease is a type-2 hypersensitivity reaction against the basal epithelial membrane of the conjunctiva.

In particular, conjunctival involvement is critical since the autoantibody-induced inflammation results in conjunctival scarring that may even progress after the inflammatory process has halted and leads to visual impairment and blindness.

Early diagnosis and appropriate treatment are of paramount importance to avoid inflammatory and infectious complications, as well as possible visual loss. Ocular MMP management is aimed at controlling the immune-mediated inflammatory disease preventing fibrosis and progression of the disease.

Georgoudis, P. et al, "Ocular Mucous Membrane Pemphigoid: Current State of Pathophysiology, Diagnostics and Treatment", Ophthalmol. Ther. (2019) 8, 5-17, discloses that a stepladder approach is used to select immunosuppressive agents and to escalate treatment, depending on disease severity (mild, moderate, severe). The medications used are dapsone, sulphapyridine, sulphasalazine, azathioprine (AZA), methotrexate (MTX), mycophenolate mofetil (MMF), cyclophosphamide, and short courses of oral steroids.

CD20 monoclonal antibodies, TNFα inhibitors, and intravenous immunoglobulin (IVIg) are used to treat disease in patients non-responsive to conventional immunosuppressants.

The therapeutic mainstay are high-dose systemic corticosteroids supplemented with potentially corticosteroid sparing agents such as azathioprine, mycophenoles, dapsone, antibiotics with anti-inflammatory activity such as doxycycline, high-dose-intravenous immunoglobulins, and the anti-CD20 antibody rituximab.

The current treatment armamentarium is frequently not effective and associated with severe adverse events.

Thus, there is still a high need for effective and safe therapies.

Several possible mechanisms have been proposed underlying the development of antibody-mediated diseases MMP.

CXCL8 (interleukin-8, IL-8) is an endogenous chemotactic factor produced by most nucleated cells such as fibroblasts, macrophages, endothelial and epithelial cells.

As reported, the biological activity of CXCL8 is mediated by the interaction of CXCL8 with CXCR1 and CXCR2 membrane receptors belonging to the family of seven transmembrane receptors and expressed on the surface of human neutrophils and of several types of T-cells (L. Xu et al., J. Leukocyte Biol., 57, 335, 1995). Although CXCR1 activation is known to play a crucial role in CXCL8-mediated chemotaxis, it has been supposed that also CXCR2 activation could play a pathophysiological role in chronic inflammatory diseases. Different CXCL8 inhibitors have been developed and are well known to the skilled man.

WO2000/024710 discloses N-(2-aryl-propionyl)-sulfonamides, having inhibitory activity of neutrophils chemotaxis and degranulation induced by interleukin-8, and their use in the prevention and treatment of tissue damage due to the exacerbate recruitment of polymorphonuclear neutrophils (leukocytes PMN) at the inflammatory sites, in particular in the treatment of psoriasis, rheumatoid arthritis, ulcerative colitis, acute respiratory insufficiency, idiopathic fibrosis, glomerulonephritis.

WO2005/090295 discloses (R)-2-[4-(trifluoromethanesulfonyloxy)phenyl]propionic acid derivatives, which are used as inhibitors of the chemotaxis of polymorphonucleate and mononucleate cells, particularly in the treatment of neutrophils-dependent pathologies. The use of said compounds in the treatment of psoriasis, ulcerative colitis, melanoma, angiogenesis, chronic obstructive pulmonary disease (COPD), bullous pemphigo, rheumatoid arthritis, idiopathic fibrosis, glomerulonephritis and in the prevention and treatment of damages caused by ischemia and reperfusion is also disclosed.

WO2010/031835 discloses 2-aryl-propionic acids and derivatives, substituted in the 4 position by 2-amino-heterocycles as good CXCL8-induced chemotaxis inhibitors, useful in the prevention and treatment of tissue damage due to the exacerbated recruitment of polymorphonucleated neutrophils (PMN leukocytes) at inflammation sites. The use of said compounds in the treatment of transient cerebral ischemia, damages caused by ischemia and reperfusion, bullous pemphigo, rheumatoid arthritis, idiopathic fibrosis and glomerulonephritis is also disclosed.

The present invention is aimed at providing an effective treatment of MMP, in particular ocular and oral MMP.

### SUMMARY OF THE INVENTION

The invention is directed to CXCL8 inhibitors for use in the prevention and/or treatment of ocular mucous membrane pemphigoid (OcMMP) and/or oral mucous membrane pemphigoid in a subject.

The invention is also directed to pharmaceutical compositions comprising a CXCL8 inhibitor and at least one pharmaceutically acceptable excipient or carrier for use in the prevention and/or treatment of ocular mucous membrane pemphigoid and/or oral mucous membrane pemphigoid in a subject.

The invention is also directed to a method of preventing and/or treating ocular mucous membrane pemphigoid and/or oral mucous membrane pemphigoid in a subject, which comprises administering an effective amount of one or more CXCL8 inhibitor compounds of the invention to a subject in need thereof. The invention is also directed to the use of the claimed CXCL8 inhibitors in the manufacture of a medicament for the prevention and/or treatment of ocular mucous membrane pemphigoid and/or oral mucous membrane pemphigoid in a subject.

### BRIEF DESCRIPTION OF FIGURES

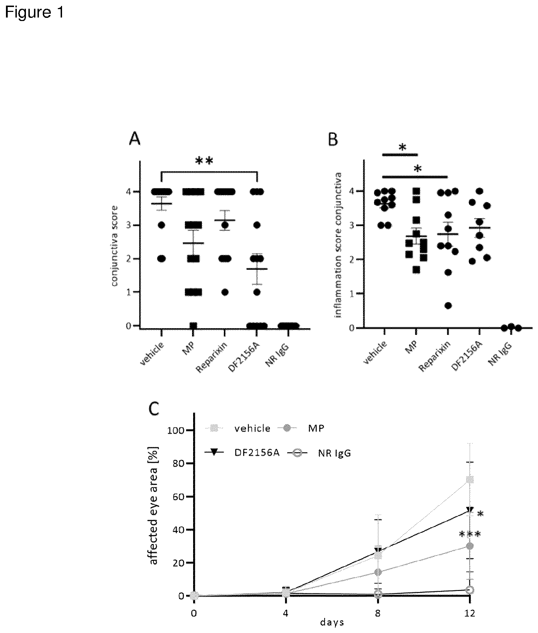

**Figure 1** shows the effects of treatment with DF2156A or reparixin, by topical ophthalmic administration, or methylprednisolone (MP), by intraperitoneal injection, in an experimental MMP mouse model at day 12 after the initial anti-mLAMα3 IgG injection, compared to control animals treated with vehicle, by topical ophthalmic administration (vehicle). Animals treated with IgG isolated from normal rabbit serum injected s.c. (NR IgG), were used as negative control. In details:
   Panel A shows the effect of treatment with DF2156A, reparixin or methylprednisolone on palpebral conjunctival split formation at day 12, expressed as conjunctiva score.
   Data are presented as means +/- standard deviation. On day 12 there is a statistically significant difference between the vehicle group and DF2156A (p=0.0016). Data are based on 12-14 mice per group, except for NR IgG (n=10). The asterix indicates statistical significance (ANOVA with Holm Sidaks method for multiple comparisons).
   Panel B shows semi-quantification of subepithelial inflammatory infiltrate based on hematoxylin and eosin (H&E) stained biopsies of the palpebral conjunctiva in mice treated with DF2156A, reparixin or methylprednisolone. On day 12 there is a statistically significant difference between the vehicle group and reparixin (p=0.0474) as well as MP (p=0.0419). Animals treated with IgG isolated from normal rabbit serum injected s.c. (NR IgG) were used as negative control. Data are presented as means +/- standard deviation. Data are based on 7-10 mice per group, except for NR IgG (n=3). The asterix indicates statistical significance (ANOVA with Holm Sidaks method for multiple comparisons).
   Panel C shows the macroscopic affected eye area evaluated at days 0, 4, 8 and 12 in mice treated with DF2156A or methylprednisolone (MP). Extent of macroscopic affected eye area is expressed as percentage. Data are presented as means +/- standard deviation. On day 12 there is a statistically significant difference between the vehicle group and DF2156A (p=0.0165) as well as MP (p<0.0001). Animals treated with IgG isolated from normal rabbit serum injected s.c. (NR IgG) were used as negative control. Data are based on 12-14 mice per group, except for NR IgG (n=10) and presented as means +/- standard deviation. Mixed-effects analysis with Dunnett's method for multiple comparisons.

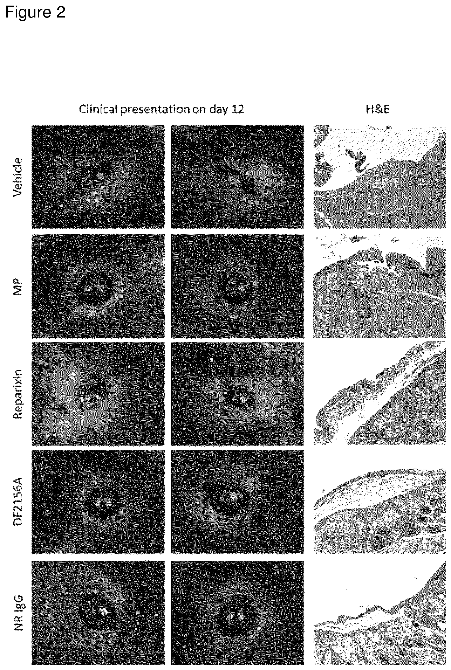

**Figure 2** shows representative clinical presentations of the eyes and H&E stained sections of the palpebral conjunctiva obtained 12 days after the initial anti-mLAMα3 IgG injection in mice treated with DF2156A or reparixin, by topical ophthalmic administration, or methylprednisolone (MP), by intraperitoneal injection.

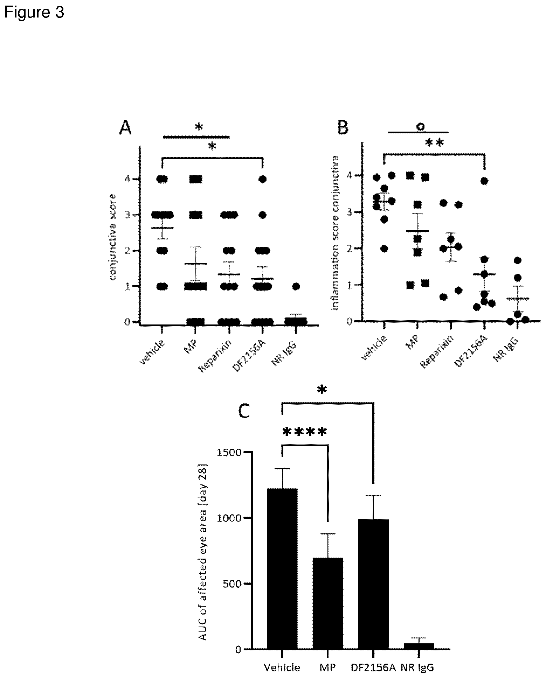

**Figure 3** shows the reduction of split formation in the palpebral conjunctiva in experimental MMP mice at day 28 after the initial anti-mLAMcc3 IgG injection, treated with DF2156A or reparixin, by topical ophthalmic administration, or methylprednisolone (MP), by intraperitoneal injection. In details:
   Panel A shows the effect of treatment with DF2156A, reparixin or methylprednisolone on palpebral conjunctival split formation on day 28, expressed as conjunctiva score, compared to vehicle.
   Animals treated with IgG isolated from normal rabbit serum injected s.c. (NR IgG) were used as negative control. Data are presented as means +/- standard deviation. On day 28 there is a statistically significant difference between the vehicle group and DF2156A (p=0.0229) as well as reparixin (p=0.0412). Data are based on 11-14 mice per group, except for NR IgG (n=9). The asterix indicates statistical significance (ANOVA with Holm Sidaks method for multiple comparisons).
   Panel B shows the semi-quantification of subepithelial inflammatory infiltrate based on hematoxylin and eosin (H&E) stained biopsies of the palpebral conjunctiva in mice treated with DF2156A, reparixin or methylprednisolone (MP), compared to vehicle. Animals treated with IgG isolated from normal rabbit serum injected s.c. (NR IgG) were used as negative control. Data are presented as means +/- standard deviation. On day 28 there is a statistically significant difference between the vehicle group and DF2156A (p=0.0031). Furthermore, reparixin significantly decreased the inflammatory infiltrate when only compared to vehicle treated mice (p=0.0280). Data are based on 7-9 mice per group, except for NR IgG (n=5). The asterix indicates statistical significance based on ANOVA with Dunnetts method for multiple comparisons and the open circle indicates statistical significance by ANOVA with uncorrected Fishers LSD method (no correction for multiple comparisons).
   Panel C shows the extent of affected eye area expressed as the area under the curve (AUC) over 28 days in mice treated with MP or DF2156A, compared to vehicle. Animals treated with IgG isolated from normal rabbit serum injected s.c. (NR IgG) were used as negative control. Data are presented as means +/standard deviation and based on 11-14 mice per group, except for NR IgG (n=9). On day 28 there is a statistically significant difference between the vehicle group and DF2156A (p=0.0113) as well as MP (p<0.0001). The asterix indicates statistical significance (ANOVA with Dunnett method for multiple comparisons).

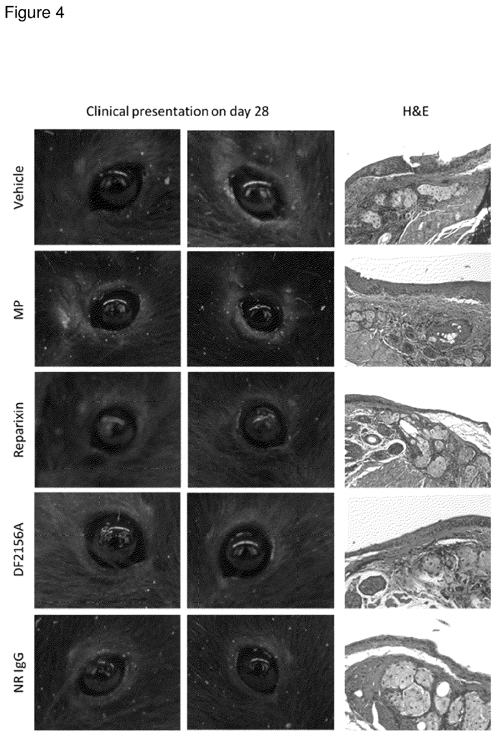

**Figure 4** shows representative clinical presentations of the eyes and H&E stained sections of the palpebral conjunctiva obtained 28 days after the initial anti-mLAMα3 IgG injection in mice treated with DF2156A or reparixin, by topical ophthalmic administration, or methylprednisolone (MP), by intraperitoneal injection.

### DETAILED DESCRIPTION OF THE INVENTION

It has been surprisingly found that the CXCL8 inhibitors are effective in the prevention and/or treatment of ocular mucous membrane pemphigoid (OcMMP) and oral mucous membrane pemphigoid.

Accordingly, the invention is directed to CXCL8 inhibitors for use in the prevention and/or treatment of ocular mucous membrane pemphigoid (OcMMP) and/or oral mucous membrane pemphigoid in a subject.

The terms "treatment" and "prevention" as used herein refer to the eradication/amelioration or prevention/delay in onset, respectively, of the disorder being treated or of one or more of the symptoms associated thereof, notwithstanding the fact that the patient may still be afflicted with the underlying disorder.

The term "CXCL8 inhibitors" in accordance with the present invention means any compound able to inhibit the biological activity of CXCL8. Preferably, a CXCL8 inhibitor according to the present invention is a CXCL8 receptor(s) inhibitor.

Preferably said CXCL8 receptor(s) inhibitor is a CXCR1- or a CXCR1/2-inhibitor, which inhibits the activity of CXCL8 mediated by CXCR1 receptor or mediated by both the CXCR1 and CXCR2 receptors.

Said CXCL8 receptor(s) inhibitor preferably inhibits the binding of CXCL8 to the CXCR1 receptor or to both the CXCR1 and CXCR2 receptors, or prevents or blocks the intracellular signaling activated by the binding of CXCL8 to the CXCR1 receptor or to both the CXCR1 and CXCR2 receptors.

According to a preferred embodiment, the CXCL8 receptor(s) inhibitor is an antagonist of the CXCR1 receptor or an antagonist of both CXCR1 and CXCR2 receptors.

According to another preferred embodiment, the CXCL8 receptor(s) inhibitor is an allosteric inhibitor or an orthosteric antagonist of CXCR1 receptor or of both CXCR1 and CXCR2 receptors.

Alternatively, the CXCL8 receptor(s) inhibitor preferably binds to CXCL8, thus preventing its binding to its receptors.

Said CXCL8 receptor inhibitor(s) is preferably able to inhibit in an in-vitro assay at least 60%, preferably at least 70%, more preferably at least 80%, even more preferably at least 90% of PMNs chemotaxis induced by 1 nM CXCL8 at a concentration equal or below 500 nM, preferably below 100 nM.

More preferably, the CXCL8 receptor(s) inhibitor according to the invention has an IC₅₀ value towards CXCR1 receptor in the low nanomolar range, preferably lower than 10 nanomolar, more preferably in the range 0.02-5 nanomolar.

According to a further preferred embodiment, said CXCL8 inhibitor is selected from small molecules, peptides and antibodies, more preferably it is a small molecule.

The term "small molecule" refers to an organic compound having a molecular weight of 900 Daltons or lower.

CXCL8 inhibitors, in particular CXCL8 receptor(s) inhibitors, as defined above, are well known in the art.

To date, several CXCL8 inhibitors, such as small molecules, peptides and antibodies, have been disclosed, many of which are currently undergoing clinical trials or are used in therapy (Jie Jack, Expert Opinion Ther. Patents, 2001, 11(12), Chao J. et al., Bioorganic & Medicinal Chemistry Letters 17, 2007, p. 3778-3783, Busch-Petersen J. Current Topics in Medicinal Chemistry, 2006, 6, p. 1345-135, Allegretti et al, Immunology Letters 2012, Vol. 145, p. 68-78).

Preferably, the CXCL8 inhibitor according to the invention is selected from the group comprising (or consisting of):
- the anti-CXCL-8 antibodies ABCream, BMS-986253 and ABX-IL-8;
- RP-72, PAC-G-31-P, SCH-N,
- Navarixin, having formula: 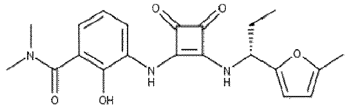
- SX-517, having formula: 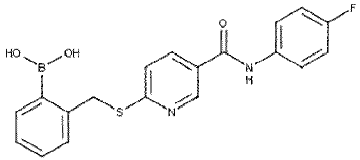
- SX-576, having formula: 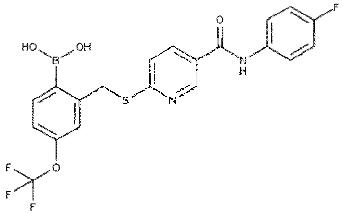
- SX-682, having formula: 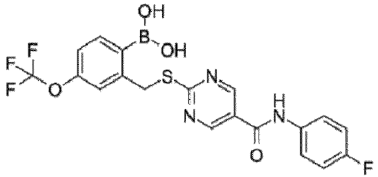
- compounds having formula: 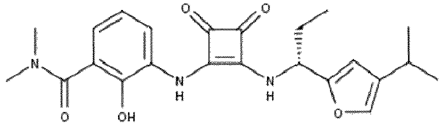 or 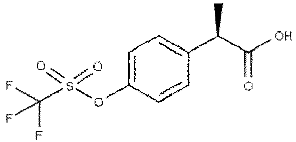 or 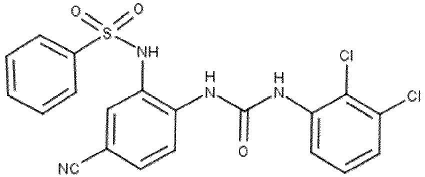 and
- 5-[3-(2-Fluorophenyl)ureido]-1-(2-hydroxypropyl)-1H-pyrazole-4-carboxylic acid ethyl ester
- 5-[3-(3-Fluorophenyl)ureido]-1-(2-hydroxypropyl)-1H-pyrazole-4-carboxylic acid ethyl ester
- 3-[2-[1(R)-(4-Bromofuran-2-yl)propylamino]-3,4-dioxo-1-cyclobutenylamino]-2-hydroxy-N,N-dimethylbenzamide
- 3-[2-[1(R)-(4-Chlorofuran-2-yl)propylamino]-3,4-dioxo-1-cyclobutenylamino]-2-hydroxy-N,N-dimethylbenzamide
- Trifluoromethanesulfonic acid 4-[1(R)-(N-isopropylcarbamoyl)ethyl]phenyl ester
- 2-Hydroxy-3-[4-[1(R)-(4-isopropylfuran-2-yl)propylamino]-1-oxo-1,2,5-thiadiazol-3-ylamino]-N,N-dimethylbenzamide
- 3-(2-Chlorophenylamino)-7-nitro-4H-1,2,4-benzothiadiazin-5-ol 1,1-dioxide
- 1-[3-[4-[3-(4-Fluorophenyl)isoxazol-5-yl]phenoxy]propyl]-4-methylpiperazine
- N-(2-[(2,3-Difluorobenzyl)sulfanyl]-6-[[(2R,3S)-3,4-dihydroxybutan-2-yl]oxy]pyrimidin-4-yl)azetidine-1-sulfonamide; and
- the compounds of formula (I) and (II) described hereinbelow.

According to one preferred embodiment, said CXCL8 inhibitor have general formula (I): 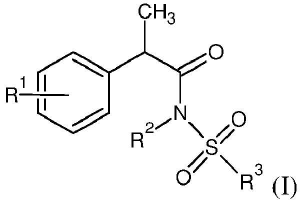 wherein
R¹ is selected from a linear or branched C₁-C₆ alkyl, benzoyl, phenoxy, and trifluoromethanesulfonyloxy;
R² is selected from hydrogen and a linear or branched C₁-C₃ alkyl; and
R³ is a linear or branched C₁-C₆ alkyl or trifluoromethyl,
or a pharmaceutically acceptable salt thereof.

According to the present invention, "C₁-C₆-alkyl" represents a linear or branched alkyl chain containing 1 to 6 carbon atoms.

R¹ is preferably selected from benzoyl, isobutyl, and trifluoromethanesulfonyloxy. R¹ is preferably linked to the phenyl ring in 3- or 4-position. According to the most preferred embodiment, R¹ is 3-benzoyl, 4-isobutyl or 4-trifluoromethanesulfonyloxy.

R² is preferably selected from hydrogen or methyl.

R³ is preferably selected from linear or branched C₁-C₆ alkyl, more preferably from linear of branched C₁-C₃ alkyl. According to the most preferred embodiment, R³ is methyl.

The chiral carbon of the compounds of formula (I) is in the RS or R configuration, more preferably it is in the R configuration.

Particularly preferred compounds of formula (I) according to the invention are selected from:
- 2-(4-isobutylphenyl)propionyl methansulfonamide, preferably R-(-)-2-(4-isobutylphenyl)propionyl methansulfonamide (also known as reparixin) and pharmaceutically acceptable salts thereof, preferably the lysine salt thereof, and
- 2-[(4-trifluoromethanesulfonyloxy)phenyl]-N-methanesulfonyl propionamide, preferably R(-)-2-[(4-trifluoromethanesulfonyloxy)phenyl]-N-methanesulfonyl propionamide and pharmaceutically acceptable salts thereof, in particular the sodium salt thereof (also known as ladarixin or DF2156A). Compounds of formula (I) are described in WO2000/024710A1 and WO2005/090295A2, which also disclose their method of synthesis. According to one preferred embodiment, said CXCL8 inhibitor have general formula (II): 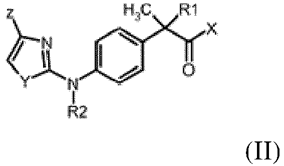 wherein:
   R1 is hydrogen or CH₃;
   X is OH;
   R2 is hydrogen or linear C₁-C₄ alkyl,
   Y is a heteroatom selected from S, O and N,
   Z is selected from linear or branched C₁-C₄ alkyl, linear or branched C₁-C₄ alkoxy, halo C₁-C₃ alkyl and halo C₁-C₃ alkoxy,
   or pharmaceutically acceptable salts thereof.

Preferably, the chiral carbon of the compounds of formula (II) is in the R or S configuration, more preferably it is in the S configuration.

A particularly preferred compound of formula (II) according to the invention is 2-(4-{[4-(trifluoromethyl)-1,3-thiazol-2-yl]amino}phenyl)propanoic acid, preferably (2S)-2-(4-{[4-(trifluoromethyl)-1,3-thiazol-2-yl] amino} phenyl) propanoic acid or the sodium salt thereof.

Another particularly preferred compound of formula (II) according to the invention is 2-methyl-2-(4- {[4-(trifluoromethyl)-1,3-thiazol-2-yl] amino} phenyl)propanoic acid (DF2726Y) or a pharmaceutically acceptable salt thereof, preferably the sodium salt (DF2726A).

Compounds of formula (II) are described in WO2010/031835A2, which also discloses their method of synthesis.

Preferred CXCL8 inhibitors according to the invention are DF2156A or reparixin, more preferably the CXCL8 inhibitor is DF2156A.

Preferably, the CXCL8 inhibitor for use according to the present invention is administered topically to the surface of the eye of a subject. Preferably, the CXCL8 inhibitor for use according to the present invention is formulated in form of eyedrops.

As will be discussed in the experimental section, the present inventors have shown that topical application of a CXCL8 inhibitor to the surface of the eye results in amelioration of both ocular and oral MMP.

The invention is also directed to a pharmaceutical composition comprising a CXCL8 inhibitor, as described above, and at least one pharmaceutically acceptable excipient or carrier for use in the prevention and/or treatment of ocular mucous membrane pemphigoid and/or oral mucous membrane pemphigoid in a subject.

Preferably, the pharmaceutical composition is an ophthalmic composition suitable for topical application to the surface of the eye.

Accordingly, the invention is further directed to an ophthalmic composition comprising a therapeutically effective amount of a CXCL8 inhibitor, as described above, and at least one ophthalmologically acceptable excipient or carrier.

An "ophthalmologically acceptable excipient" is an inert excipient which allows delivery of a medicament to the eye and/or eyelids, to treat an ocular disease or condition without deleterious effects on the eye.

According to an embodiment, said ophthalmic composition may be a liquid, eye drop composition for topical administration to the anterior segment of the eye. Said liquid composition may be in form of a solution, emulsion, or suspension. Said liquid composition may include micelles.

In one embodiment, the liquid composition is an aqueous composition. Preferably, the liquid composition is an aqueous eye drop composition.

Preferably, said liquid composition comprises ophthalmologically acceptable excipients selected from ophthalmologically acceptable viscosity enhancers, penetration enhancers, buffering agents, osmolarity regulators, preservatives and surfactants.

Viscosity enhancers have the function to increase viscosity of the composition and to improve its retention in the conjunctival sac and are preferably selected from cellulose derivatives, preferably hydroxymethylcellulose, hydroxyethylcellulose, hydroxypropylmethylcellulose, methylcellulose; polyvinylpyrrolidone and gelling agents, preferably gellan, xanthan gum and carbopol-974.

Penetration enhancers have the function of enhancing drug permeability across ocular membranes and are preferably selected from cyclodextrins, chelating agents, crown ethers, bile acids and bile salts.

Buffering agents have the function of providing and maintaining the correct pH of the formulation to be compatible for use in the eye, preferably at a pH comprised between 6 and 8. The preferred buffer is phosphate buffer, but other buffers capable of maintaining the pH within the desired range, especially buffers suitable for ophthalmic use, are also included.

Osmolarity regulators are salts able to make the liquid composition isotonic with ocular fluids. The preferred salt is sodium chloride (NaCl) but other biologically acceptable salts may be used, such as for instance potassium chloride (KCl), calcium chloride (CaCl₂) and magnesium chloride (MgCl₂) and their admixtures.

Preservatives inhibit microbial activity. Suitable preservatives include for instance quaternary ammonium compounds such as benzalkonium chloride, cetyltrimethylammonium bromide and cetylpyridinium chloride.

Surfactants have the function of making the composition stable and are preferably selected from polysorbates such as Tween 80, poloxamers such as Pluronics F68 or proteins such as serum albumin.

Said liquid, eye drop composition can be part of a kit comprising the composition, a container for holding the composition and a drop dispenser.

A "therapeutically effective amount" according to the present invention means an amount sufficient to achieve treatment or prevention of the disease. Determination of the effective amounts is well within the capability of those skilled in the art based upon the achievement of a desired effect. An effective amount will depend on factors including, but not limited to, the weight of a subject and/or the degree of the disease or unwanted condition from which a subject suffers.

The invention relates also to the use of the above CXCL8 inhibitors in the manufacture of a medicament for the prevention and/or treatment of ocular mucous membrane pemphigoid and/or oral mucous membrane pemphigoid. The invention is also directed to a method of preventing and/or treating ocular mucous membrane pemphigoid and/or oral mucous membrane pemphigoid, which comprises administering an effective amount of one or more CXCL8 inhibitor of the invention to a subject in need thereof.

The invention is further illustrated by the following example.

### EXAMPLE

A study was designed to investigate and show the effect of pharmacological inhibition of CXCR1/2 exerted by the compound DF2156A and reparixin in a mouse model of MMP.

### Materials and methods

### Test compounds

- Ladarixin Sodium Salt (DF2156A) (Dompé S.p.A.)
- Reparixin Lysine Salt (Dompé S.p.A.)
- Vehicle (solvent, Dompé S.p.A.) - (positive control)
- Methylprednisolone (MP) (sourced by UKSH, Lübeck - Supplier Sanofi) - (reference treatment)

### Animal model - Mice

Adult C57Bl/6 (B6) mice (male and female) aged at least 6 weeks old were used. Animals were maintained on a 12-h light-dark cycle at the animal facility of the University of Lübeck. Mice were held under SPF conditions and fed acidified drinking water and standard chow ad libitum. Protocols were approved by the Animal Rights Commission of the Ministry of Agriculture and Environment, Schleswig-Holstein.

### Generation, isolation and characterization of anti-mLAMα3 IgG

For production of anti-mLAMα3 IgG, New Zealand White rabbits were immunized subcutaneously with 250µg of an equimolar mixture of the 2 purified recombinant proteins (aa1656-1985 and aa2756-3330 of murine Laminin alpha3 chain, produced in E. coli as disclosed by Heppe, E.N., et al., J Invest Dermatol, 2017, 137, 1709-1718) suspended in complete Freund's adjuvant. The animals were boosted twice with the same protein preparation in incomplete Freund's adjuvant. Immune sera were obtained at regular intervals and characterized by IF microscopy on cryosections of murine skin. IgG from rabbits immunized with recombinant fragments of murine mLAMα3 and from non-immunized rabbits was purified by affinity chromatography using protein G sepharose affinity chromatography (Amersham Biosciences, Heidelberg, Germany). Reactivity of IgG fractions was analyzed by IF microscopy on murine skin (Sitaru et al., J Immunol 2006, 177: 3461-8). In addition, each batch of anti-mLAMα3 IgG was characterized *in vivo* regarding its capability to induce experimental MMP in C57Bl/6 (B6) mice (WP1.1). Form this experiment, the amount of antibodies was determined, that leads to moderate experimental MMP; i.e. the amount needed to induce disease development with moderate involvement of the conjunctiva (i.e. score of 1-3 in a maximum of 50% of the animals on day 12) in the model of antibody transfer-induced MMP. For this purpose, the intensity of the separation of the conjunctiva is determined histologically. The body surface area affected should be between 3-8% at the concentration used. The conjunctiva score was set histologically post-mortem and was in a range of 0-4, depending on the intensity of the separation of the conjunctiva. The amount needed was determined to be 5 mg/injection of anti-mLAMα3 IgG.

### Study design

### Induction of experimental MMP by repetitive injections of anti-mLAMα3 IgG and treatment protocol

To test DF2156A at 0.5% and reparixin at 0.5% effects on the severity of conjunctival involvement in experimental MMP, the disease was induced by repetitive subcutaneous (s.c.) injections of anti-mLAMα3 IgG (5mg/mouse) on alternating days (0, 2, (...) and 10) into adult B6 mice, which were treated throughout the entire experiment twice a day via eyedrops. Three eyedrops per eye were given twice daily. Mice treated with solvent via eyedrops or methylprednisolone (MP) via i.p. injections were used as treatment controls. Mice s.c. injected with IgG isolated from normal rabbit serum (NR IgG) served as negative control.

### Primary endpoint - Evaluation of conjunctival lesions

The primary endpoint of this experiment was the extent of conjunctival lesions as determined by lesional histopathology (H&E stain) on day 12 and 28 following an established scoring system (Heppe, E.N., et al., J Invest Dermatol, 2017, 137, 1709-1718). In detail, biopsies from the palpebral conjunctiva were taken on day 12 and day 28 and paraffine embedded. 4.5µm thick sections from 3 different depths of the biopsies were cut in triplicate and H&E stained for further quantification. Among the H&E stained histologies those with a palpebral conjunctival-epithelium with more than 1000µm were used for quantification. For this score the length of the split formation (split = epithelia separation from the underlying dermal structures) is measured. The split length is categorized to a score of 0-4 with no split = 0, less than 100µm = 1, less than 200µm = 2, less than 300µm = 3, more or equal 300µm = 4. Splits that occurred at the end of the tissue were excluded, because they were most likely to be of artificial nature due to the cutting. The longest split out of 9 possible sections is decisive for the final score.

The endpoint was analyzed on day 12 in the following groups:
▪ normal rabbit IgG (n=10)
▪ Anti-mLAMα3 IgG + solvent (positive control) (n=14)
▪ Anti-mLAMα3 IgG + MP (reference treatment) (n=13)
▪ Anti-mLAMα3 IgG + DF2156A (n=13)
▪ Anti-mLAMα3 IgG + reparixin (n=14)

The endpoint was analyzed on day 28 in the following groups:
▪ normal rabbit IgG (n=9)
▪ Anti-mLAMα3 IgG + solvent (positive control) (n=11)
▪ Anti-mLAMα3 IgG + MP (reference treatment) (n=11)
▪ Anti-mLAMα3 IgG + DF2156A (n=14)
▪ Anti-mLAMα3 IgG + reparixin (n=12)

The experiments were performed at two independent time points for each day 12 and day 28, including 7 mice/group and 5 mice/normal rabbit IgG.

### Severity of oral lesions

The extent of oral lesions was determined by endoscopy (Videomed, München, Germany) on day 28 following an established scoring system. In detail, each affected mouth-quarter of the mouse is counted as one point if there are lesions/blisters/crusts/erosions. The quarters are defined as: left buccal mucosa, right buccal mucosa, hypopharynx, tongue. The maximum score is 4.

### Statistical analysis

For statistical analysis GraphPad Prism (version 8.4.3) was used. For comparison of treatment effects in multiple groups ANOVA was used. To isolate the group or groups that differ from others, Dunnett's or Holm Sidaks multiple comparison were used when appropriate.

### Results

### 1. Local application of DF2156A and reparixin reduced split formation in the palpebral conjunctiva in experimental MMP

Injection of rabbit anti-mLAMα3 IgG into adult B6 mice persistently leads to induction of experimental MMP within 4-8 days after the first IgG injection.

After 12 days of twice daily local application of the drugs as eyedrops a significant reduction of the conjunctival split formation for the treatment groups of DF2156A compared to mice that received the vehicle was determined **(****figure 1A****).** This is also reflected by a reduced subepithelial inflammatory cell infiltrate by semi-quantification of H&E stained palpebral conjunctival biopsies taken on day 12 of mice treated with DF2156A compared to those biopsies of mice that received the vehicle **(****figure 1B****).**

The effect of DF2156A administered topically on the extent of affected eye area was also measured in parallel with that of MP administered systemically (i.p, once daily). A reduction of the affected eye area was observed for both treatments on day 12 **(****figure 1C****).** In mice injected with NR-IgG no conjunctival lesions were observed compared to the other groups on day 12 **(****figure 2****).** Treatment over 28 days twice daily with local application via eyedrops lead to a significant reduction of palpebral conjunctival split formation in mice receiving DF2156A or reparixin **(****figure 3A****).** This is also reflected in a decreased subepithelial inflammatory infiltrate by semi-quantification of H&E stained biopsies of the palpebral conjunctiva **(****figure 3B****).** The effect of DF2156A administered topically on the extent of affected eye area was also measured in parallel with that of MP administered systemically (i.p, once daily). A reduction of the area was observed for both treatments on day 28 **(****figure 3C****).** Mice that just received NR-IgG from healthy rabbits did not show conjunctival lesions compared to the other groups on day 28 **(****figure 4****).**

### Conclusions

The data obtained show that:
- DF2156A treatment resulted in an improvement with regard to the conjunctival split formation already on day 12 and until day 28, and with regard to the inflammatory infiltrate on day 28;
- Reparixin treatment reduced the inflammatory infiltration of the palpebral conjunctiva after 12 and 28 days and also reduced the conjunctiva score after 28 days.

On the basis of the data obtained, both DF2156A and reparixin administered topically twice a day resulted in an overall benefit for diseased mice, with a slower onset of the therapeutic effect for reparixin (Figures 2 and 4).

### 2. Local application of DF2156A reduces oral lesions in experimental MMP

Treatment with DF2156A decreased severity of oral involvement after 28 days of eyedrops application, with a statistically significant difference between the vehicle group and DF2156A (p=0.0152).

## Claims

1. A CXCL8 inhibitor for use in the prevention and/or treatment of ocular mucous membrane pemphigoid and/or oral mucous membrane pemphigoid in a subject.

2. A CXCL8 inhibitor for use as claimed in claim 1, wherein said CXCL8 inhibitor is administered topically to the surface of the eye of the subject.

3. A CXCL8 inhibitor for use according to claim 1 or claim 2, which is a CXCL8 receptor(s) inhibitor selected from a CXCR1- or a CXCR1/2-inhibitor.

4. A CXCL8 inhibitor for use according to any one of claims 1 to 3, which is an antagonist of the CXCR1 receptor or an antagonist of both CXCR1 and CXCR2 receptors.

5. A CXCL8 inhibitor for use according to any one of claims 1 to 4, which is a CXCL8 receptor(s) inhibitor selected from an allosteric inhibitor or an orthosteric antagonist of CXCR1 receptor or of both CXCR1 and CXCR2 receptors.

6. A CXCL8 inhibitor for use according to any one of claims 1 to 5, having general formula (I): 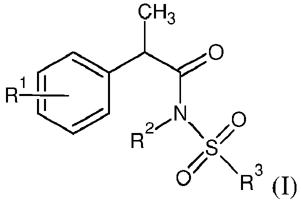 wherein
R¹ is selected from a linear or branched C₁-C₆ alkyl, benzoyl, phenoxy, and trifluoromethanesulfonyloxy;
R² is selected from hydrogen and a linear or branched C₁-C₃ alkyl; and
R³ is a linear or branched C₁-C₆ alkyl or trifluoromethyl,
or a pharmaceutically acceptable salt thereof.

7. A CXCL8 inhibitor for use according to claim 6, wherein the chiral carbon of the compounds of formula (I) is in the R configuration.

8. A CXCL8 inhibitor for use according to any one of claims 1 to 5, having general formula (II): 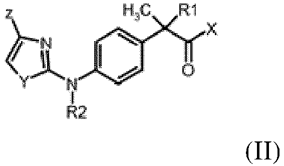 wherein:
R1 is hydrogen or CH₃;
X is OH;
R2 is hydrogen or linear C₁-C₄ alkyl,
Y is a heteroatom selected from S, O and N,
Z is selected from linear or branched C₁-C₄ alkyl, linear or branched C₁-C₄ alkoxy, halo C₁-C₃ alkyl and halo C₁-C₃ alkoxy,
or pharmaceutically acceptable salts thereof.

9. A CXCL8 inhibitor for use according to claim 8, wherein the chiral carbon of the compounds of formula (II) is in the S configuration.

10. A CXCL8 inhibitor for use according to any one of claims 1 to 9, wherein said inhibitor is selected from the group consisting of:
- the anti-CXCL-8 antibodies ABCream, BMS-986253 and ABX-IL-8;
- RP-72, PAC-G-31-P, SCH-N,
- Navarixin, having formula: 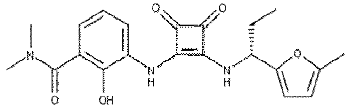
- SX-517, having formula: 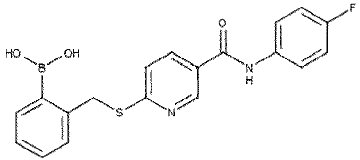
- SX-576, having formula: 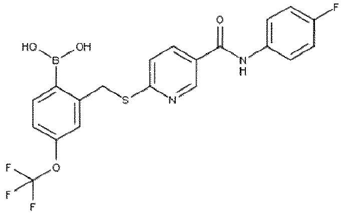
- SX-682, having formula: 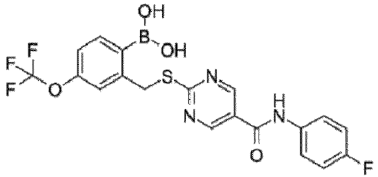
- compounds having formula: 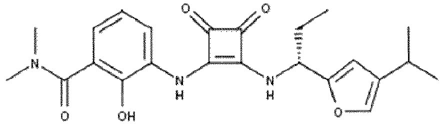 or 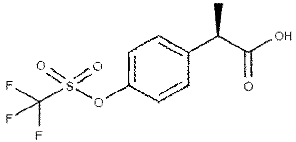 or 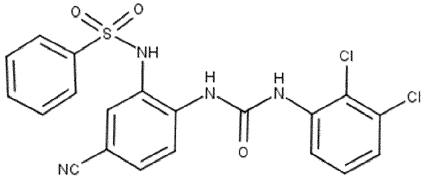 and
- 5-[3-(2-Fluorophenyl)ureido]-1-(2-hydroxypropyl)-1H-pyrazole-4-carboxylic acid ethyl ester
- 5-[3-(3-Fluorophenyl)ureido]-1-(2-hydroxypropyl)-1H-pyrazole-4-carboxylic acid ethyl ester
- 3-[2-[1(R)-(4-Bromofuran-2-yl)propylamino]-3,4-dioxo-1-cyclobutenylamino]-2-hydroxy-N,N-dimethylbenzamide
- 3-[2-[1(R)-(4-Chlorofuran-2-yl)propylamino]-3,4-dioxo-1-cyclobutenylamino]-2-hydroxy-N,N-dimethylbenzamide
- Trifluoromethanesulfonic acid 4-[1(R)-(N-isopropylcarbamoyl)ethyl]phenyl ester
- 2-Hydroxy-3-[4-[1(R)-(4-isopropylfuran-2-yl)propylamino]-1-oxo-1,2,5-thiadiazol-3-ylamino]-N,N-dimethylbenzamide
- 3-(2-Chlorophenylamino)-7-nitro-4H-1,2,4-benzothiadiazin-5-ol 1,1-dioxide
- 1-[3-[4-[3-(4-Fluorophenyl)isoxazol-5-yl]phenoxy]propyl]-4-methylpiperazine
- N-(2-[(2,3-Difluorobenzyl)sulfanyl]-6-[[(2R,3S)-3,4-dihydroxybutan-2-yl]oxy]pyrimidin-4-yl)azetidine-1-sulfonamide; and
- the compounds of formula (I) and (II) as defined in claims 6 to 9.

11. A CXCL8 inhibitor for use according to claim 6 or 7, having general formula (I) which is selected from:
- 2-(4-isobutylphenyl)propionyl methansulfonamide, preferably R-(-)-2-(4-isobutylphenyl)propionyl methansulfonamide and pharmaceutically acceptable salts thereof, preferably the lysine salt thereof, and
- 2-[(4-trifluoromethanesulfonyloxy)phenyl]-N-methanesulfonyl propionamide, preferably R(-)-2-[(4-trifluoromethanesulfonyloxy)phenyl]-N-methanesulfonyl propionamide and pharmaceutically acceptable salts thereof, in particular the sodium salt thereof.

12. A CXCL8 inhibitor for use according to claim 11, which is the sodium salt of R(-)-2-[(4-trifluoromethanesulfonyloxy)phenyl]-N-methanesulfonyl propionamide.

13. A CXCL8 inhibitor for use according to claim 8 or 9, having general formula (II) which is 2-(4-{ [4-(trifluoromethyl)-1,3-thiazol-2-yl]amino}phenyl)propanoic acid, preferably (2S)-2-(4-{[4-(trifluoromethyl)-1,3-thiazol-2-yl] amino} phenyl) propanoic acid or the sodium salt thereof.

14. An ophthalmic composition comprising a therapeutically effective amount of a CXCL8 inhibitor as defined in claims 2-13 and at least one ophthalmologically acceptable excipient or carrier.
